# FASCICULE DE BREVET EUROPEEN

(11) **EP 0 804 173 B1**
(45) Date de publication et mention de la délivrance du brevet: **28.10.1998**
(21) Numéro de dépôt: 95917394.9
(22) Date de dépôt: 13.04.1995
(51) Int. Cl.: A61K 9/50, A61K 9/16

(54) **MICROSPHERES BIODEGRADABLES A LIBERATION CONTROLEE ET LEUR PROCEDE DE PREPARATION**
BIOLOGISCH ABBAUBARE MIKROSPHÄREN MIT GESTEUERTER FREIGABE UND VERFAHREN ZU DEREN HERSTELLUNG
CONTROLLED RELEASE BIODEGRADABLE MICROSPHERES AND METHOD OF PREPARATION

(30) Priorité: 15.04.1994 FR 9404511
(43) Date de publication de la demande: 05.11.1997
(73) Titulaire: PIERRE FABRE MEDICAMENT, 92100 Boulogne (FR)
(72) Inventeur: AROLA, Rosa, 08015 Barcelone (ES); ASIN, Miguel Angel, 08290 Cerdanyola (ES); FERRET, Eulalia, 08820 El Prat de Llobregat (ES); GOUTAY, Eric, 31650 Auzielle (FR); PEREZ, Amadeo, 08028 Barcelone (ES); TARIN, Pere, 08202 Sabadell (ES)
(74) Mandataire: Ahner, Francis
(86) Numéro de dépôt international: FR9500485
(87) Numéro de publication internationale: WO9528149

(56) Documents cités:
- EP-A- 0 481 732
- WO-A-93/25191
- WO-A-93/25221
- JOURNAL OF CONTROLLED RELEASE, vol.30, no.3, Juillet 1994, AMSTERDAM (NL) pages 201 - 211, XP453418 H. SAH ET AL. 'the influence of biodegradable microcapsule formulations on the controlled release of a protein'

## Description

La présente invention a pour objet la réalisation de microsphères biodégradables à base de composés pharmacologiquement actifs hydrophiles, permettant d'assurer quelle que soit la voie d'administration retenue (par exemple la voie injectable), une libération contrôlée et soutenue de ladite substance active.

De nombreuses formes pharmaceutiques à base de polymères ou de copolymères biodégradables et biocompatibles ont déjà été décrites dans l'état de la technique antérieure. Ces diverses formes sont obtenues par des procédés dont la spécificité est toujours étroitement liée :
- à la voie d'administration souhaitée (taille, forme de la forme galénique),
- aux propriétés physico-chimiques de la molécule encapsulée notamment sa solubilité, sa thermostabilité et sa température de fusion,
- aux propriétés pharmacologiques et pharmacodynamiques de cette molécule active.

Dans la plupart des applications, les voies oculaires, dermiques, auriculaires et orales, il n'est pas nécessaire de mettre au point des formes galéniques de faibles dimensions. C'est pourquoi les procédés de fusion-extrusion, nébulisation, fluidisation, voire compression sur machine à comprimer classique, sont très largement décrits et utilisés.

Pour les formes galéniques implantables, les procédés de fabrication sont également nombreux, car la taille de l'implant ne nécessite pas de descendre très bas en granulométrie et l'on retrouve donc les procédés cités ci-dessus.

En revanche, pour les formes destinées à être administrées par la voie parentérale, les procédés de fabrication se trouvent très fortement liés aux contraintes suivantes :
- d'une part, la taille de la forme galénique, qui doit pouvoir être administrée sous forme de suspension injectable par une seringue appropriée (18 à 22 gauges). Ceci oblige à tenir compte de cette contrainte et à obtenir des microcapsules ou microsphères de taille inférieure à 150 µm,
- d'autre part, la stabilité physico-chimique de la molécule et sa solubilité (hydrosoluble, liposoluble) qui conditionne, pour les procédés en milieu liquide, le sens des émulsions simples ou multiples,
- et enfin, le profil pharmacocinétique optimal de ladite forme qui conditionne du même coup la durée de l'activité du produit.

Malgré ces contraintes, quelquefois très dures à surmonter, il existe une grande variété de procédés de microencapsulation, parmi lesquels on peut citer :
- l'émulsion-évaporation de solvant,
- l'émulsion-extraction de solvant,
- la séparation de phase,
- la nébulisation ou spray-drying,
- l'extrusion, et
- la fluidisation en lit d'air.

L'objet de la présente invention est relatif à l'élaboration de microsphères à base d'une molécule hydrophile, par un procédé du type émulsion multiple-évaporation de solvant.

De façon générale, dans un procédé de ce type par émulsion multiple E/H/E et évaporation du solvant, le principe actif hydrosoluble est solubilisé tout d'abord dans la phase interne d'une première émulsion E/H, puis, dans un second temps, cette première émulsion est à son tour émulsionnée dans une phase aqueuse externe.

Cette technique s'est avérée particulièrement avantageuse pour formuler des principes actifs très hydrosolubles pour lesquels elle donne une bonne efficacité d'encapsulation.

Le principe physique d'émulsion multiple pour encapsuler des principes actifs hydrosolubles a par exemple été décrit dans US-A-3,523,906.

La présente invention concerne un procédé de préparation de composition pharmaceutique sous forme de microsphère à libération contrôlée d'au moins un principe actif hydrosoluble, caractérisé par la succession des étapes suivantes :
- dissolution du principe actif dans une quantité appropriée d'eau,
- émulsification de la solution aqueuse de principe actif ainsi obtenue avec une solution d'au moins un copolymère matriciel du type d,l-lactide-co-glycolide dans un hydrocarbure chloré contenant en outre un agent modulateur de libération, polylactide de faible poids moléculaire, conduisant à une première émulsion microfine et homogène,
- émulsification de ladite première émulsion ainsi obtenue dans une phase aqueuse externe, contenant un agent tensio-actif,
- extraction-évaporation du solvant pour obtenir des microsphères que l'on récupère après filtration, lavage et séchage.

D'autres caractéristiques et avantages apparaîtront à la lecture de la description détaillée faite ci-après, notamment en s'appuyant sur quelques exemples de mise en oeuvre particuliers.

Cette invention consiste à élaborer des compositions pharmaceutiques, sous forme de microsphères, destinées à obtenir une libération contrôlée et soutenue d'un principe actif hydrosoluble, selon un procédé d'Emulsion Multiple E/H/E et Extraction-Evaporation du Solvant, dans lequel, selon une caractéristique particulière consistant à utiliser un système évaporateur continu constitué d'une pente de longueur adéquate sur laquelle s'écoule en couche mince la suspension de microsphères, on parvient à diminuer sa durée dans le temps.

Les principes actifs hydrosolubles (I) qui peuvent être utilisés dans le cadre de ce procédé comprennent des substances naturelles ou synthétiques douées d'une activité pharmacologique, telles que peptides, bronchiodilatateurs, antibiotiques, antidépresseurs, analgésiques, antiinflammatoires, vaccins etc. On citera particulièrement les principes actifs suivants : calcitonine, vasopressine, somatostatine, hormone de croissance, prolactine, hormone lutéinisante, ocytocine, sécrétine, salbutamol, gentamycine, tétracycline, tobramycine, amikacine, salicylate de sodium, diclofenac de sodium, amoxicilline, ampicilline, naproxen sodique, vinorelbine, vincristine, vindesine, methotrexate, cis platine, carmustine, doxorubicine, epirubicine, daunorubicine, ifosfamide, et leurs dérivés. Cette liste de principes actifs n'illustre que des produits susceptibles d'être encapsulés par ce procédé, sans toutefois en exclure d'autres.

Les concentrations utilisées dans la phase aqueuse interne dans le cadre de la présente invention sont fonction de la solubilité du principe actif dans l'eau, des caractéristiques dudit principe actif et de la libération soutenue que l'on désire obtenir. A titre d'exemple, on citera des concentrations allant de 0,01% à 95%, de préférence comprises entre 0,05 et 50%, en particulier entre 0,05 et 2,5%.

Le ou les polymère(s) matriciel(s) utilisable(s) (II) pour préparer des microsphères par E/H/E doivent, d'une part, être solubilisables dans un solvant volatil (III) approprié. A titre d'exemples de tels solvants (III), on citera les alcanes halogénés tels que le chlorure de méthylène, le chloroforme, le chloroéthane, le dichloroéthane, le trichloroéthane et le tétrachlorure de carbone, l'éthyle acétate, etc. D'autre part, le(s)dit(s) polymère(s) doivt être non miscible à l'eau et enfin, être biodégradable et biocompatible avec l'organisme. L'ample expérience acquise avec les copolymères polylactide-co-glycolide prouve que ceux-ci sont parfaitement tolérés par l'organisme, qu'ils ont une réponse inflammatoire minime, sont absorbés sans s'accumuler dans les organes vitaux et sont finalement totalement éliminés. C'est pour cela que les polymères choisis sont les polylactide-co-glycolides, en particulier les d,l-lactide-co-glycolides, dans des proportions lactique/glycolique de 85:15 et 40:60, de préférence entre 75:25 et 50:50. Ils offrent en effet une vitesse d'hydrolyse plus rapide évitant ainsi les dépôts à l'endroit de l'administration, après la libération du principe actif. L'agent modulateur de la libération (IV) est un polylactide, en particulier un d,l lactide, de faible poids moléculaire qui s'additionne au copolymère polylactide-co-glycolide en proportions quelconques.

Selon le procédé objet de la présente invention, le poids moléculaire du copolymère doit être compris entre 10 000 et 500 000, de préférence entre 10 000 et 250 000, avantageusement entre 12 000 et 34 000, tandis que pour l'agent modulateur de la libération, le poids moléculaire doit être compris entre 1 000 et 10 000, de préférence voisin de 2 000.

Les concentrations et poids moléculaires des polymères utilisés sont fonction du principe actif et de la vitesse de libération souhaitée. Selon le procédé objet de la présente invention, des concentrations appropriées des polymères présents dans la phase organique, sont comprises entre 5% et 50%.

La phase aqueuse externe (V) contient un tensioactif (VI) tel que les polyvinylalcools, les polyvinylpyrrolidones, le polyoxyéthylène monooléate de sorbitanne, le polyoxyéthylène monolaurate de sorbitanne, les poloxamers, le carboxyméthylcellulose sodique ou leurs mélanges. Les concentrations à utiliser vont de 0,05 à 25%. Dans ce procédé, le tensioactif retenu préférentiellement est un mélange de polyvinylpyrrolidone et de polyoxyéthylène mono-oléate de sorbitanne.

De façon plus détaillée, le procédé selon l'invention consiste à effectuer les étapes suivantes.

On prépare une quantité de principe actif (I) dissous dans un volume d'eau purifiée. Cette solution s'émulsionne par exemple à l'aide d'un sonicateur, dans un volume de chlorure de méthylène (III) contenant un copolymère polylactide-co-glycolide (II) et l'agent modulateur de la libération (IV), à savoir le polylactide de faible poids moléculaire. Cette première émulsion doit être microfine et homogène, ce qui permettra de répandre le principe actif sur toute la matrice polymérique, en assurant la reproductibilité des différents lots, sans avoir besoin d'utiliser des tensioactifs ni d'autres agents adjuvants. Une fois la première émulsion formée, on l'émulsionne à son tour dans une phase externe (V), formée par une solution aqueuse contenant de la polyvinylpyrrolidone et du polyoxyéthylène mono-oléate de sorbitanne (VI), sous agitation pendant une courte période. Après quoi, on dilue avec de l'eau purifiée et on fait passer la suspension sur un système évaporateur continu constitué d'une pente de longueur adéquate sur laquelle s'écoule en couche mince la suspension de microsphères, ce qui améliore l'extraction-évaporation du solvant et réduit la durée du procédé. Les microsphères sont récupérées par filtration, lavées avec de l'eau purifiée et séchées par lyophilisation.

Ce procédé, simple à réaliser et reproductible, permet d'obtenir des microsphères d'une taille adéquate pour leur administration.

Une caractéristique importante de cette invention réside dans le fait que l'addition du polylactide de faible poids moléculaire au copolymère polylactide-co-glycolide permet de moduler la libération du principe actif. Il a été constaté qu'en augmentant la proportion de polylactide de faible poids moléculaire, on augmentait la vitesse de libération du produit encapsulé (voir exemple 1 et 2) qui se trouve dans les petits réservoirs à l'intérieur des microsphères (voir exemple 7).

Selon une caractéristique additionnelle de la présente invention, le rapport du copolymère à l'agent modulateur est compris entre 10:90 et 90:10, de préférence entre 50:50 et 90:10.

De façon générale, les microsphères obtenues conformément au procédé de l'invention sont toujours inférieures à environ 250 µm, de préférence inférieures à environ 100 µm, et avantageusement comprises entre 3 et 40 µm.

La formation de la seconde émulsion est l'étape la plus critique de ce procédé, quant à l'efficacité d'encapsulation, surtout si l'on veut obtenir de petites tailles.

L'originalité du procédé selon la présente invention permet de réduire le temps total de mise en oeuvre du procédé, provoque une stabilisation précoce de la matrice polymérique et évite la perte de produit encapsulé. La vitesse de durcissement des microsphères permet une bonne efficacité d'encapsulation même en ajoutant un polylactide de faible poids moléculaire dans la solution polymérique, sans qu'il faille utiliser d'adjuvants dans la première émulsion. De plus, le procédé est mis en oeuvre à température ambiante et sous pression atmosphérique, ce qui fait que l'on évite des problèmes tels que l'altération des produits thermolabiles ou la cassure de microsphères quand on utilise le vide à l'étape d'extraction-évaporation.

Selon une autre caractéristique additionnelle de la présente invention, la proportion entre les phases de la première émulsion est comprise entre 1:100 et 25:100.

L'un des buts essentiels de cette invention est de moduler la libération du principe actif en ajustant la proportion entre le polylactide de faible poids moléculaire et le polylactide-co-glycolide pour l'élaboration des microsphères. Cette forme de modulation est facilement reproductible.

Pour garantir la reproductibilité de la libération (voir exemple 3), il est indispensable que la proportion du copolymère et de l'agent modulateur se maintienne dans la matrice polymérique. Il a été constaté expérimentalement par RMN qu'il y avait une corrélation linéaire entre le pourcentage théorique de polylactide de faible poids moléculaire incorporé et le pourcentage réel trouvé. Pour chaque niveau de polylactide de faible poids moléculaire (entre 10% et 90%) les résultats obtenus sont reproductibles (voir exemple 4).

### Exemple 1

On dissout 20 mg de tartrazine dans 1 ml d'eau purifiée. Cette solution s'émulsionne à l'aide d'un sonicateur dans 10 ml de chlorure de méthylène contenant 15% de mélange d'un copolymère, poly(d,l-lactide-co-glycolide) 50:50 avec une viscosité inhérente d'approximativement 0,4 dl/g dans du CHCl₃ et des quantités croissantes d'un agent modulateur de la libération, poly (d,l-lactide) de poids moléculaire 2000. Une fois formée la première émulsion, on l'émulsionne à son tour dans 1000 ml de solution aqueuse formée par de la polyvinylpyrrolidone à 4% et du polyoxyéthylène mono-oléate de sorbitanne à 0,25 %, sous agitation d'hélice pendant 90 secondes. Après quoi, on passe la suspension par un système évaporateur continu constitué d'une pente de longueur adéquate sur laquelle s'écoule en couche mince la suspension de microsphères. On récupère les microsphères sous filtration, on lave avec de l'eau purifiée et on sèche sous lyophilisation.

Avec ce procédé, on a élaboré des microsphères avec les proportions de polylactide de faible poids moléculaire suivantes :

| | | | | | | |
|---|---|---|---|---|---|---|
| **Exemples** | 1.1 | 1.2 | 1.3 | 1.4 | 1.5 | 1.6 |
| **%polylactide** | 0% | 10% | 20% | 30% | 40% | 50% |

### Pourcentage de tartrazine libérée à partir de microsphères en tampon phosphate à pH 7,4 et à 37°C (moyenne de deux valeurs ou plus)

| **Exemple** | **4h** | **6h** | **24h** | **48h** |
|---|---|---|---|---|
| 1.1 | 4,1 | 3,9 | 5,9 | 5,3 |
| 1.2 | 9,4 | 9,6 | 12,2 | 20,6 |
| 1.3 | 27,9 | 27,8 | 36,7 | 72,6 |
| 1.4 | 30,1 | 30,5 | 47,6 | 88,3 |
| 1.5 | 51,3 | 50,6 | 73,4 | 104,2 |
| 1.6 | 79,2 | 82,6 | 97,1 | 102,2 |

### Exemple 2

On dissout 20 mg de tartrazine dans 1 ml d'eau purifiée. Cette solution s'émulsionne à l'aide d'un sonicateur dans 10 ml de chlorure de méthylène contenant 20% de mélange d'un copolymère, poly (d,l-lactide-co-glycolide) 50:50 avec une viscosité inhérente d'approximativement 0,2 dl/g dans du CHCl₃ et des quantités croissantes d'un agent modulateur de la libération, poly (d,l-lactide) de poids moléculaire 2000. Pour l'élaboration des microsphères, on procède comme à l'exemple 1.

Avec ce procédé, on a élaboré des microsphères avec les proportions de polylactide de faible poids moléculaire suivantes :

| | | | | |
|---|---|---|---|---|
| **Exemple** | 2.1 | 2.2 | 2.3 | 2.4 |
| **%polylactide** | 0% | 10% | 20% | 40% |

### Pourcentage de tartrazine libérée à patir de microsphères en tampon phosphate à pH 7.4 et à 37°C (moyenne de deux valeurs ou plus)

| **Exemple** | **4h** | **6h** | **24h** | **48h** |
|---|---|---|---|---|
| 2.1 | 5,2 | 5,3 | 5,6 | 6,5 |
| 2.2 | 12,4 | 11,6 | 14,7 | 22,4 |
| 2.3 | 43,2 | 53,0 | 71,8 | 82,4 |
| 2.4 | 81,4 | 88,2 | 103,6 | 102,3 |

### Exemple 3

Etude de reproductibilité de la libération "in vitro" de 3 formulations des exemples 1 et 2.

### Pourcentage de tartrazine libérée à partir de microsphères en tampon phosphate à pH 7,4 et à 37°C (moyenne de deux valeurs ou plus)

| **Exemple** | **6h** | **24h** | **48h** | **72h** | **96h** |
|---|---|---|---|---|---|
| 1.4 | 32,1 | 43,6 | ----- | 97,8 | 99,1 |
| 1.4 | 30,5 | 47,6 | 88,3 | 100,1 | 101,8 |
| 2.2 | 14,8 | 18,3 | 21,7 | 27,3 | 36,8 |
| 2.2 | 11,6 | 14,7 | 22,4 | 32,0 | ----- |
| 2.3 | 53,0 | 71,8 | 82,4 | 85,1 | 89,5 |
| 2.3 | ----- | 77,8 | 82,1 | 92,8 | 94,5 |

### Exemple 4

Etude par RMN de la reproductibilité du mélange polymérique que forme la matrice de microsphères élaborées selon le procédé décrit dans les exemples 1 et 2.

| % PLA théorique | % PLA réel |
|---|---|
| 10 | 8,9 |
| 20 | 16,3 |
| | 16,9 |
| | 15,5 |
| 30 | 24,0 |
| | 24,6 |
| 40 | 33,1 |
| | 35,2 |
| | 35,0 |
| | 34,4 |
| 50 | 44,0 |
| | 42,4 |
| | 42,7 |
| 60 | 52,9 |
| | 51,5 |
| | 51,9 |
| 70 | 59,1 |
| | 60,5 |
| | 59,5 |
| 80 | 69,4 |
| | 69,8 |
| | 68,4 |
| 90 | 78,4 |
| | 77,7 |
| | 77,9 |
| PLA : polylactide de p.m. 2 000 | |

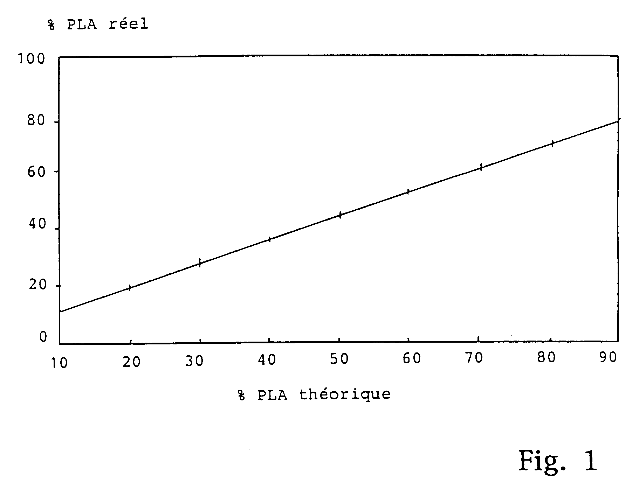

La variation des valeurs corrélées statistiquement de % PLA réel en fonction de PLA théorique est représentée sur le schéma de la Figure 1 annexée.

### Exemple 5

Elaboration de microsphères selon le procédé décrit à l'exemple 1, en utilisant la calcitonine de saumon comme principe actif.

### Exemple 5.1

On dissout 7,5 mg de calcitonine de saumon dans 1 ml d'eau purifiée et on l'émulsionne dans 10 ml de chlorure de méthylène contenant 1 500 mg de poly (d,l-lactide-co-glycolide) 50:50 d'une viscosité inhérente d'approximativement 0,4 dl/g dans du CHCl₃. Pour l'élaboration des microsphères, on procède comme à l'exemple 1.

La taille moyenne en aire, obtenue par "laser light scattering", est de 16,8µm. L'efficacité est de 92,5%.

### Exemple 5.2

On dissout 7,5 mg de calcitonine de saumon dans 1 ml d'eau purifiée et on l'émulsionne dans 10 ml de chlorure de méthylène contenant un mélange formé par 1050 mg de poly(d,l-lactide-co-glycolide) 50:50 avec une viscosité inhérente d'approximativement 0,4 dl/g dans du CHCL₃ et 450 mg d'un agent modulateur de la libération, poly(d,l-lactide) de poids moléculaire 2000. Pour l'elaboration des microsphères, on procède comme à l'exemple 1.

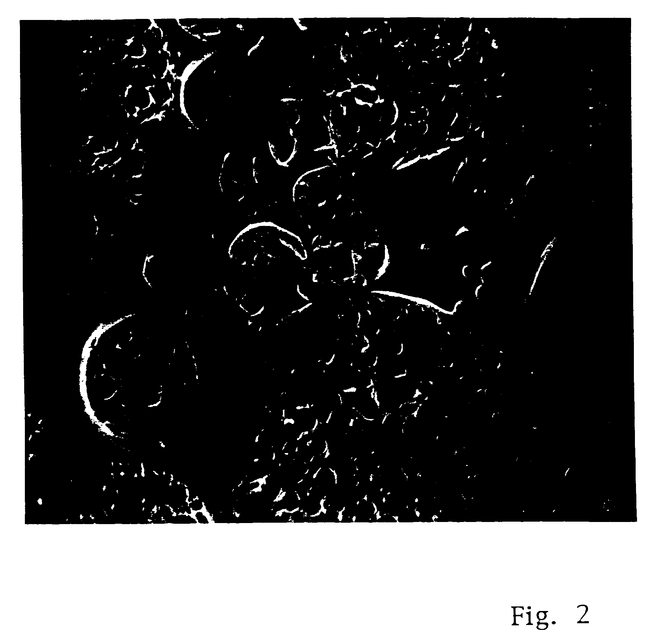

La taille moyenne en aire, obtenue par "laser light scattering", est de 22,9µm. L'efficacité est de 102,5%. Une photographie des microsphères sélectionnées par cryofracture, obtenue au microscope électronique à balayage, est représentée à la figure 2 annexée.

### Exemple 5.3

On dissout 7,5 mg de calcitonine de saumon dans 1 ml d'eau purifiée et on l'émulsionne dans 10 ml de chlorure de méthylène contenant un mélange formé par 900 mg de poly(d,l-lactide-co-glycolide) 50:50 avec une viscosité inhérente d'approximativement 0,4 dl/g dans du CHCL₃ et 600 mg d'un agent modulateur de la libération, poly(d,l-lactide) de poids moléculaire 2000. Pour l'élaboration des microsphères, on procède comme à l'exemple 1.

La taille moyenne en aire, obtenue par "laser light scattering", est de 17,6µm. L'efficacité est de 98,5%.

### Exemple 5.4

On dissout 10 mg de calcitonine de saumon dans 1 ml d'eau purifiée et on l'émulsionne dans 10 ml de chlorure de méthylène contenant 2000 mg de poly(d,l-lactide-co-glycolide) 50:50 avec une viscosité inhérente d'approximativement 0,2 dl/g dans du CHCL₃. Pour l'élaboration des microsphères, on procède comme à l'exemple 1.

La taille moyenne en aire, obtenue par "laser light scattering", est de 30,4µm. L'efficacité est de 92,6%.

### Exemple 5.5

On dissout 10 mg de calcitonine de saumon dans 1 ml d'eau purifiée et on l'émulsionne dans 10 ml de chlorure de méthylène contenant un mélange composé de 1400 mg de poly(d,l-lactide-co-glycolide) 50:50 avec une viscosité inhérente d'approximativement 0,2 dl/g dans du CHCL₃ et 600 mg d'un agent modulateur de la libération, poly(d,l-lactide) de poids moléculaire 2000. Pour l'élaboration des microsphères, on procède comme à l'exemple 1.

La taille moyenne en aire, obtenue par "laser light scattering", est de 36,2µm. L'efficacité est de 106,5%.

### Exemple 5.6

On dissout 10 mg de calcitonine de saumon dans 1 ml d'eau purifiée et on l'émulsionne dans 10 ml de chlorure de méthylène contenant un mélange composé de 1200 mg de poly(d,l-lactide-co-glycolide) 50:50 avec une viscosité inhérente d'approximativement 0,2 dl/g dans du CHCL₃ et 800 mg d'un agent modulateur de la libération, poly(d,l-lactide) de poids moléculaire 2000. Pour l'élaboration des microsphères, on procède comme à l'exemple 1.

La taille moyenne en aire, obtenue par "laser light scattering", est de 39,8µm. L'efficacité est de 86,4%.

### Exemple 5.7

On dissout 51 mg de calcitonine de saumon dans 1 ml d'eau purifiée et on l'émulsionne dans 10 ml de chlorure de méthylène contenant un mélange composé de 1200 mg de poly(d,l-lactide-co-glycolide) 50:50 avec une viscosité inhérente d'approximativement 0,2 dl/g dans du CHCL₃ et 800 mg d'un agent modulateur de la libération, poly(d,l-lactide) de poids moléculaire 2000. Pour l'élaboration des microsphères, on procède comme à l'exemple 1.

La taille moyenne en aire, obtenue par "laser light scattering", est de 32,2µm. L'efficacité est de 109,4%.

### Exemple 5.8

Une solution de 100 µl de calcitonine de saumon à 12,5% est émulsionnée dans 10 ml de chlorure de méthylène contenant 2250 mg de poly(d,l-lactide-co-glycolide) 50:50 d'une viscosité inhérente d'approximativement 0,2 dl/g dans du CHCl₃ et 250 mg d'un agent modulateur de la libération, poly (d,l-lactide) de poids moléculaire 2000, à l'aide d'un sonicateur. Une fois formée la première émulsion, on l'émulsionne à son tour dans 300 ml d'une solution aqueuse de polyoxyéthylène mono-oléate de sorbitanne à l'aide d'un homogénéisateur Ultraturrax pendant 3 minutes. Après quoi, on passe la suspension par un système évaporateur continu constitué d'une pente de longueur adéquate sur laquelle s'écoule en couche mince la suspension de microsphères et on sèche les microsphères par lyophilisation.

La taille moyenne en aire, obtenue par "laser light scattering", est de 8µm. L'efficacité est de 72,4%.

### Exemple 5.9

On dissout 7,5 mg de calcitonine de saumon dans 1 ml d'eau purifiée et on l'émulsionne dans 10 ml de chlorure de méthylène contenant un mélange composé de 150 mg de poly(d,l-lactide-co-glycolide) 50:50 avec une viscosité inhérente d'approximativement 0,4 dl/g dans du CHCL₃ et 1350 mg d'un agent modulateur de la libération, poly(d,l-lactide) de poids moléculaire 2000. Pour l'élaboration des microsphères, on procède comme à l'exemple 1.

La taille moyenne en aire, obtenue par "laser light scattering", est de 19,7µm. L'efficacité est de 95,3%.

### Exemple 5.10

On dissout 10 mg de calcitonine de saumon dans 1 ml d'eau purifiée et on l'émulsionne dans 10 ml de chlorure de méthylène contenant un mélange composé de 200 mg de poly(d,l-lactide-co-glycolide) 50:50 avec une viscosité inhérente d'approximativement 0,2 dl/g dans du CHCL₃ et 1800 mg d'un agent modulateur de la libération, poly(d,l-lactide) de poids moléculaire 2000. Pour l'élaboration des microsphères, on procède comme à l'exemple 1.

La taille moyenne en aire, obtenue par "laser light scattering", est de 33,1µm. L'efficacité est de 98,5%.

### Exemple 6

Effet produit par l'inclusion de quantités croissantes de poly(d,l-lactide) de poids moléculaire 2000, dans la matrice polymérique de poly(d,l-lactide-co-glycolide) 50:50, de viscosité inhérente de 0.4 dl/g dans du CHCl₃, sur la libération "in vitro" de la calcitonine de saumon.

### Pourcentage de calcitonine de saumon libérée à partir de microsphères en tampon phosphates à pH 7,4 et à 37°C.

| **Exemple** | **1j** | **3j** | **4j** | **9j** | **10j** |
|---|---|---|---|---|---|
| 5.1 | ----- | 0 | 0 | 0,7 | 1,0 |
| 5.2 | ----- | 1,8 | 2,3 | 5,0 | 5,0 |
| 5.3 | 5,3 | 6,4 | 7,9 | 10,4 | 11,4 |

Effet que produit l'inclusion de quantités croissantes de poly(d,l-lactide) de poids moléculaire 2000, dans la matrice polymérique de poly(d,l-lactide-co-glycolide) 50:50, de viscosité inhérente de 0,2 dl/g dans du CHCl₃, sur la libération "in vitro" de la calcitonine de saumon.

### Pourcentage de calcitonine de saumon libérée à partir de microsphères en tampon phosphates à pH 7,4 et à 37°C.

| **Exemple** | **1j** | **4j** | **6j** | **8j** | **10j** |
|---|---|---|---|---|---|
| 5.4 | 0,8 | 0,8 | 0,8 | 1,2 | 2,2 |
| 5.5 | 5,7 | 12,0 | 14,2 | 17,0 | 18,0 |
| 5.6 | 8,9 | 16,1 | 17,7 | 18,9 | 20,9 |

Enfin, à titre d'exemples additionnels, on mentionnera ci-après quelques principes actifs qui ont été incorporés dans des microsphères préparées selon le procédé décrit dans les exemples 1 et 2, et qui ont conduit aux temps de libération indiqués ci-après.

| **Principe actif** | **Temps de libération** |
|---|---|
| Amoxicilline sodique | 1 jour à 1 semaine |
| Naproxen sodique | 1 jour à 1 mois |
| Salbutamol sulfate | 1 semaine à 1 mois |
| Vinorelbine | 1 semaine à 1 mois |
| Vincristine | 1 semaine à 1 mois |
| Vindesine | 1 semaine à 1 mois |
| Methotrexate | 1 semaine à 1 mois |
| Cis platine | 1 semaine à 1 mois |
| Carmustine | 1 semaine à 1 mois |
| Doxorubicine | 1 semaine à 1 mois |
| Epirubicine | 1 semaine à 1 mois |
| Daunorubicine | 1 semaine à 1 mois |
| Ifosfamide | 1 semaine à 1 mois |

## Revendications

1. Procédé de préparation de composition pharmaceutique sous forme de microsphère à libération contrôlée d'au moins un principe actif hydrosoluble, caractérisé par la succession des étapes suivantes :
- dissolution du principe actif dans une quantité appropriée d'eau,
- émulsification de la solution aqueuse de principe actif ainsi obtenue avec une solution d'au moins un copolymère matriciel du type d,l-lactide-co-glycolide dans un hydrocarbure chloré contenant en outre un agent modulateur de libération polylactide de faible poids moléculaire, conduisant à une première émulsion microfine et homogène,
- émulsification de ladite première émulsion ainsi obtenue dans une phase aqueuse externe, contenant un agent tensio-actif,
- extraction-évaporation du solvant pour obtenir des microsphères que l'on récupère après filtration, lavage et séchage.

2. Procédé selon la revendication 1, caractérisé en ce que le principe actif est choisi parmi les substances suivantes : calcitonine, vasopressine, somatostatine, hormone de croissance, prolactine, hormone lutéinisante, ocytocine, sécrétine, salbutamol, gentamycine, tétracycline, tobramycine, amikacine, salicylate de sodium, diclofenac de sodium, amoxicilline, ampicilline, naproxen sodique, vinorelbine, vincristine, vindesine, methotrexate, cis platine, carmustine, doxorubicine, epirubicine, daunorubicine, ifosfamide, et leurs dérivés.

3. Procédé selon l'une des revendications 1 et 2, caractérisé en ce que dans ledit copolymère matriciel la proportion lactique : glycolique est comprise entre 75 : 25 et 50 : 50.

4. Procédé selon l'une des revendication 1 à 3, caractérisé en ce que le poids moléculaire du copolymère matriciel est compris entre 10 000 et 250 000, de préférence entre 12 000 et 34 000.

5. Procédé selon l'une des revendications 1 à 4, caractérisé en ce que le poids moléculaire du polylactide modulateur est compris entre 1 000 et 10 000, de préférence voisin de 2 000.

6. Procédé selon l'une des revendications 1 à 5, caractérisé en ce que la proportion entre le copolymère matriciel et le polylactide modulateur est comprise entre 10:90 et 90:10, de préférence entre 50:50 et 90:10.

7. Procédé selon l'une des revendications 1 à 6, caractérisé en ce que l'on dissout le principe actif hydrosoluble dans de l'eau pour former la phase aqueuse interne, sans l'addition d'aucune substance qui retienne le principe actif, ni d'agents stabilisateurs de l'émulsion, ni aucune opération destinée à augmenter la viscosité; on émulsionne cette phase dans une phase organique formée par un mélange dudit copolymère et dudit agent modulateur de la libération, constitué d'un homopolymère du polylactide de bas poids moléculaire et dudit hydrocarbure chloré, pour préparer la première émulsion (E/H); on ajoute celle-ci à une troisième phase aqueuse qui contient un tensioactif, pour préparer la seconde émulsion (E/H/E); et on élimine rapidement le solvant à température ambiante et sous pression atmosphérique pour produire les microsphères.

8. Procédé selon la revendication 7, caractérisé en ce que ladite première émulsion est microfine et homogène.

9. Procédé selon la revendication 7, caractérisé en ce que la proportion entre les phases de la première émulsion est comprise entre 1:100 et 25:100.

10. Procédé selon la revendication 7, caractérisé en ce que ledit solvant est rapidement éliminé, à température ambiante et sous pression atmosphérique, selon un système évaporateur continu constitué d'une pente de longueur adéquate sur laquelle s'écoule en couche mince la suspension de microsphères.

11. Procédé selon les revendications 1 à 10, caractérisé en ce que ledit hydrocarbure chloré est le chlorure de méthylène.

12. Procédé selon les revendications 1 à 11, caractérisé en ce que ledit tensioactif est un mélange de polyvinylpyrrolidone et polyoxyéthylène mono-oléate de sorbitanne.

13. Microsphères obtenues par la mise en oeuvre du procédé selon l'une des revendications 1 à 12.

14. Microsphères selon la revendication 13, caractérisées en ce que leur taille est de 250 µm ou moins, de préférence de 125 µm ou moins, et de façon encore préférentielle de 50 µm ou moins.

## Claims

1. Process for preparing a pharmaceutical composition in the form of microspheres affording controlled release of at least one water-soluble active principle, characterized by the following succession of steps:
- dissolution of the active principle in a suitable amount of water,
- emulsification of the aqueous solution of active principle thereby obtained with a solution of at least one matrix copolymer of the dl-lactide-co-glycolide type in a chlorinated hydrocarbon containing, in addition, a release-modulating agent which is a low molecular weight polylactide, leading to a first, microfine and homogeneous emulsion,
- emulsification of said first emulsion thereby obtained in an external aqueous phase containing a surfactant,
- extraction-evaporation of the solvent to obtain microspheres which are recovered after filtration, washing and drying.

2. Process according to Claim 1, characterized in that the active principle is chosen from the following substances: calcitonin, vasopressin, somatostatin, growth hormone, prolactin, luteinizing hormone, oxytocin, secretin, salbutamol, gentamicin, tetracycline, tobramycin, amikacin, sodium salicylate, diclofenac sodium, amoxicillin, ampicillin, naproxen sodium, vinorelbine, vincristine, vindesine, methotrexate, cisplatin, carmustine, doxorubicin, epirubicin, daunorubicin, ifosfamide and derivatives thereof.

3. Process according to one of Claims 1 and 2, characterized in that, in said matrix copolymer, the lactic/glycolic ratio is between 75:25 and 50:50.

4. Process according to one of Claims 1 to 3, characterized in that the molecular weight of the matrix copolymer is between 10,000 and 250,000, preferably between 12,000 and 34,000.

5. Process according to one of Claims 1 to 4, characterized in that the molecular weight of the modulating polylactide is between 1000 and 10,000, preferably in the region of 2000.

6. Process according to one of Claims 1 to 5, characterized in that the ratio of the matrix copolymer to the modulating polylactide is between 10:90 and 90:10, preferably between 50:50 and 90:10.

7. Process according to one of Claims 1 to 6, characterized in that the water-soluble active principle is dissolved in water to form the internal aqueous phase, without the addition of any substance which might retain the active principle or of any agents that stabilize the emulsion, or any operation intended for increasing the viscosity; this phase is emulsified in an organic phase composed of a mixture of said copolymer and said release-modulating agent consisting of a low molecular weight homopolymer of polylactide and said chlorinated hydrocarbon, to prepare the first (W/O) emulsion; the latter is added to a third aqueous phase which contains a surfactant, to prepare the second (W/O/W) emulsion; and the solvent is removed rapidly at room temperature and at atmospheric pressure to produce the microspheres.

8. Process according to Claim 7, characterized in that said first emulsion is microfine and homogeneous.

9. Process according to Claim 7, characterized in that the ratio between the phases of the first emulsion is between 1:100 and 25:100.

10. Process according to Claim 7, characterized in that said solvent is rapidly removed, at room temperature and at atmospheric pressure, according to a continuous evaporation system consisting of an incline of appropriate length over which the suspension of microspheres flows in a thin layer.

11. Process according to Claims 1 to 10, characterized in that said chlorinated hydrocarbon is methylene chloride.

12. Process according to Claims 1 to 11, characterized in that said surfactant is a mixture of polyvinylpyrrolidone and polyoxyethylenesorbitan monooleate.

13. Microspheres obtained by carrying out the process according to one of Claims 1 to 12.

14. Microspheres according to Claim 13, characterized in that they are 250 µm or less, preferably 125 µm or less and still more preferably 50 µm or less in size.

## Patentansprüche

1. Verfahren zur Herstellung eines pharmazeutischen Zusammensetzung in Form von Mikrosphären zur kontrollierten Freisetzung von mindestens einem wasserlöslichen aktiven Wirkstoff, charakterisiert durch die Aufeinanderfolge der folgenden Schritte:
- Lösen des aktiven Wirkstoffs in einer geeigneten Menge Wasser,
- Emulgieren der so erhaltenen wäßrigen Lösung des aktiven Wirkstoffs mit einer Lösung von mindestens einem Matrix-Copolymer vom Typ d,l-Lactid-co-glycolid in einem chlorierten Kohlenwasserstoff, enthaltend darüber hinaus als Freisetzungs-Modulator ein Polylactid mit geringem Molekulargewicht, was zu einer ersten mikrofeinen und homogenen Emulsion führt,
- Emulgieren dieser so erhaltenen ersten Emulsion in einer externen wäßrigen Phase, die ein oberflächenaktives Mittel enthält,
- Extraktion-Verdampfen des Lösungsmittels, wobei Mikrosphären erhalten werden, die man nach Filtration, Waschen und Trocknen isoliert.

2. Verfahren nach Anspruch 1, dadurch charakterisiert, daß der aktive Wirkstoff unter den folgenden Substanzen ausgewählt wird: Calcitonin, Vasopressin, Somatostatin, Wachstumshormon, Prolactin, luteinisierendes Hormon, Oxytocin, Secretin, Salbutamol, Gentamycin, Tetracyclin, Tobramycin, Amikacin, Natriumsalicylat, Natriumdiclofenac, Amoxicillin, Ampicillin, Natriumnaproxen, Vinorelbin, Vincristin, Vindesin, Methotrexat, cis-Platin, Carmustin, Doxorubicin, Epirubicin, Daunorubicin, Ifosfamid, und ihren Derivaten.

3. Verfahren nach einem der Ansprüche 1 und 2, dadurch charakterisiert, daß im Matrix-Copolymer das Verhältnis Lactose : Glykol zwischen 75 : 25 und 50 : 50 beträgt.

4. Verfahren nach einem der Ansprüche 1 bis 3, dadurch charakterisiert, daß das Molekulargewicht des Matrix-Copolymeren zwischen 10000 und 250000, bevorzugt zwischen 12000 und 34000 liegt.

5. Verfahren nach einem der Ansprüche 1 bis 4, dadurch charakterisiert, daß das Molekulargewicht des Polylactid-Modulators zwischen 1000 und 10000, bevorzugt in der Nähe von 2000 liegt.

6. Verfahren nach einem der Ansprüche 1 bis 5, dadurch charakterisiert, daß das Verhältnis zwischen Matrix-Copolymerem und Polylactid-Modulator zwischen 10 : 90 und 90 : 10, bevorzugt zwischen 50 : 50 und 90 : 10 liegt.

7. Verfahren nach einem der Ansprüche 1 bis 6, dadurch charakterisiert, daß man den wasserlöslichen aktiven Wirkstoff zur Bildung der internen wäßrigen Phase in Wasser löst, weder unter Zugabe irgend einer weiteren Substanz, die den aktiven Wirkstoff zurückhält, noch von Emulsionsstabilisatoren, noch irgend einer Operation zur Erhöhung der Viskosität; man diese Phase in einer organischen Phase emulgiert, die gebildet wird durch Mischung des Copolymeren und des Freisetzungsmodulators, aufgebaut aus einem Homopolymeren von Polylactid mit niedrigem Molekulargewicht und chloriertem Kohlenwasserstoff, zur Herstellung der ersten Emulsion (E/H); man dazu eine dritte wäßrige Phase gibt, die ein oberflächenaktives Mittel enthält, zur Herstellung der zweiten Emulsion (W/O/W); und man schnell das Lösungsmittel bei Raumtemperatur und unter Atmosphärendruck zur Herstellung der Mikrosphären entfernt.

8. Verfahren nach Anspruch 7, dadurch charakterisiert, daß die erste Emulsion mikrofein und homogen ist.

9. Verfahren nach Anspruch 7, dadurch charakterisiert, daß das Verhältnis zwischen den Phasen der ersten Emulsion zwischen 1 : 100 und 25 : 100 liegt.

10. Verfahren nach Anspruch 7, dadurch charakterisiert, daß das Lösungsmittel schnell bei Raumtemperatur und unter Atmosphärendruck entfernt wird in einem kontinuierlichen Verdampfersystem, das aus einer schiefen Ebene geeigneter Länge besteht, auf der eine dünne Schicht der Suspension der Mikrosphären fließt.

11. Verfahren nach den Ansprüche 1 bis 10, dadurch charakterisiert, daß der chlorierte Kohlenwasserstoff Methylenchlorid ist.

12. Verfahren nach den Ansprüche 1 bis 11, dadurch charakterisiert, daß der oberflächenaktive Stoff eine Mischung von Polyvinylpyrrolidon und Sorbitanpolyoxyethylenmonooleat ist.

13. Mikrosphären, die durch Durchführung des Verfahrens nach einem der Ansprüche 1 bis 12 erhalten werden.

14. Mikrosphären nach Anspruch 13, dadurch charakterisiert, daß ihre Größe 250 µm oder weniger, bevorzugt 125 µm oder weniger und noch bevorzugter 50 µm oder weniger beträgt.
